# EUROPEAN PATENT APPLICATION

(11) **EP 4 205 668 A1**
(43) Date of publication of application: **05.07.2023**
(21) Application number: 22216849.4
(22) Date of filing: 28.12.2022
(51) Int. Cl.: A61B 17/12

(54) **SYSTEMS FOR EMBOLIC IMPLANT DEPLOYMENT**

(30) Priority: 29.12.2021 US 202117564764
(71) Applicant: DePuy Synthes Products, Inc., Raynham, MA 02767 (US)
(72) Inventor: BLUMENSTYK, David, Raynham, 02767 (US); SOLAUN, Daniel, Raynham, 02767 (US); SIDDIQUI, Masood, Raynham, 02767 (US)
(74) Representative: Carpmaels & Ransford LLP

(57) **Abstract**

An aneurysm treatment system can include an embolic implant, a delivery system, and a handle member that are collectively designed so that the combination of the handle member and the delivery system can be used as a deployment apparatus for the embolic implant. A proximal extension of the delivery tube can be placed inside a handle channel of handle member and the handle member can be manipulated to cause the proximal extension to bend to a predetermined angle with respect to the delivery tube, thereby separating the proximal extension from the delivery tube at a disconnection feature between the proximal extension and the delivery tube. The delivery tube includes a lumen having a pull wire that runs through the delivery tube and into the proximal extension. The proximal extension, pull wire, and handle member can be translated proximally in relation to the delivery tube to deploy the embolic implant.

## Description

### FIELD OF INVENTION

The present invention relate to aneurysm treatment devices and more particularly, to delivery systems for embolic implants.

### BACKGROUND

Numerous intravascular implant devices are known in the field. Many are deployed mechanically, via systems that combine one or more catheters and wires for delivery. Examples of implants that can be delivered mechanically include embolic elements, stents, grafts, drug delivery implants, flow diverters, filters, stimulation leads, sensing leads, or other implantable structures delivered through a microcatheter. Some obstetric and gastrointestinal implants may also be implanted via similar systems that combine one or more catheters and wires. Devices that may be released or deployed by mechanical means vary greatly in design but can employ a similar delivery catheter and wire system. Many such catheter-based delivery systems include a wire for retention of the implant in the catheter until the time for release of the device. These systems are then actuated by retracting or pulling the wire relative to the catheter. Such a wire is referred to herein as a "pull wire".

To pull the wire proximally to deploy the implant, a physician can use one of many known deployment apparatuses. Such mechanical deployment apparatuses are typically separate from the delivery system and have moving parts for gripping the pull wire and for moving the pull wire proximally. Additionally, traditional deployment apparatuses are difficult for physicians to grip due to the small size of the pull wire. Deployment methods and apparatuses that do not require auxiliary components and/or complex moving parts can simplify treatment procedures and reduce cost. Accordingly, there is a need for simplified embolic implant deployment apparatuses, including an integrated deployment handle that facilitates the gripping and retracting of a pull wire to deploy an implant.

### SUMMARY

Disclosed herein are various exemplary systems, devices, and methods of the present disclosure that can address the above needs. Examples can generally include a system for delivery of an embolic implant that includes a delivery tube, a proximal extension, a handle member, an elongated member, an embolic coil, and a disconnection feature that are collectively designed so that the system can be sued as a deployment apparatus for the embolic coil. The delivery tube can include a lumen, a proximal end, and a distal end. The proximal extension may be positioned approximate the proximal end of the delivery tube and can include a proximal end and a distal end. The proximal end of the proximal extension may be disposed within a handle channel of the handle member. The proximal end of the proximal extension may extend into the handle channel of the handle member through an aperture of the handle member. The handle member can include a handle portion that, when depressed, is configured to selectively engage the handle member to the proximal extension and bend the proximal extension to a predetermined bend angle with respect to the proximal end of the delivery tube. The elongated member can be disposed within the lumen of the delivery tube. The embolic coil can be attached to the delivery tube approximate the distal end of the delivery tube. The embolic coil can be configured to detach from the delivery tube upon proximal translation of the elongated member. The disconnection feature may fix a position of the proximal extension in relation to the delivery tube and be configured to break at the predetermined bend angle to allow the proximal translation of the proximal extension and elongated member in relation to the delivery tube.

The predetermined bend angle that causes the disconnection feature to break apart may be approximately 60 degrees as measured between the proximal extension and the proximal end of the delivery tube.

The proximal extension can be detachable from the delivery tube in response to the disconnection feature breaking. The proximal extension and the handle member, when selectively engaged to each other, can be configured to translate proximally as a single unit. The elongated member can be movable to exit the proximal end of the delivery tube in response to the proximal translation of the proximal extension.

The delivery tube can include a distal portion of a hypotube. The proximal extension can include a proximal extension of the hypotube, and the disconnection feature can be disposed between the distal portion of the hypotube and the proximal portion of the hypotube.

The disconnection feature can include circumferential laser cut openings in the hypotube.

The disconnection can be configured to cause the hypotube to break at the disconnection feature when the handle portion is depressed and bends the proximal extension to the predetermined bend angle.

The proximal extension can include a tube that has a lumen therethrough. The elongated member can be at least partially disposed within the lumen of the tube of the proximal extension and at least partially disposed within the lumen of the delivery tube.

An example embolic implantation assembly can include a delivery tube, a proximal extension, a handle member, a disconnection feature, an embolic implant, and a pull wire. The delivery tube can include a lumen therethrough, a proximal end, and a distal end. The proximal extension can extend proximally from the proximal end of the delivery tube. The proximal extension can be configured to pass through an aperture of the handle member. The handle member can be configured to selectively engage the proximal extension at a handle channel of the handle member. The handle member can include a handle slider that is configured to slide proximally along a slider path, thereby selectively engaging the handle member to the proximal extension and bending the proximal extension to a predetermined bend angle with respect to the proximal end of the delivery tube. The disconnection feature can be disposed between the proximal extension and the proximal end of the delivery tube and can join the proximal extension to the delivery tube. The disconnection feature, when in a closed configuration can have a position fixed in relation to the delivery tube. The disconnection feature can be configured to open into an open configuration to allow the proximal translation of the proximal extension in relation to the delivery tube in response to being bent to the predetermined bend angle caused by sliding the handle slider along the slider path. The embolic implant attached to the delivery tube can be approximate the distal end of the delivery tube. The pull wire can be disposed within the lumen of the delivery tube and configured to move proximally to detach the embolic implant from the delivery tube when the proximal extension is translated proximally in relation to the delivery tube.

The proximal extension can be detachable from the delivery tube at the disconnection feature. The proximal extension and handle member, when selectively engaged, can be configured to translate proximally as a single unit. The pull wire can be movable to exit the proximal end of the delivery tube in response to the proximal translation of the proximal extension in relation to the delivery tube.

The delivery tube can include a distal portion of a hypotube. The proximal extension can include a proximal portion of the hypotube, and the disconnection feature can be disposed between the distal portion of the hypotube and the proximal portion of the hypotube.

The disconnection feature can include circumferential laser cut openings in the hypotube.

The predetermined bend angle that causes the disconnection feature to open into the open configuration can be approximately 60 degrees between the proximal extension and the proximal end of the delivery tube.

The proximal extension can include a tube that has a lumen therethrough. The pull wire is at least partially disposed within the lumen of the tube of the proximal extension and at least partially disposed within the lumen of the delivery tube.

The embolic implant can include an embolic coil.

An example method of deploying an embolic implant can include the steps of manipulating a handle member engaged to a proximal extension that extends from a proximal end of a delivery tube and into an aperture of the handle member in order to position an embolic implant that is attached at a distal end of the delivery tube. The handle member can include a handle channel that is engaged to the proximal extension at a disconnection feature of the proximal extension; disconnecting the delivery tube from the proximal extension by depressing a handle portion of the handle member against the proximal extension; and moving the proximal extension and handle member as a single unit in a proximal direction in relation to the delivery tube, thereby causing a pull wire affixed to the proximal extension to move in the proximal direction in relation to the delivery tube, and thereby causing the embolic implant to detach from the distal end of the delivery tube.

The method can include moving the pull wire to exit the proximal end of the delivery tube in response to moving the selectively engaged handle member and proximal extension in the proximal direction in relation to the delivery tube.

The delivery tube can include a distal portion of a hypotube. The proximal extension can include a proximal portion of the hypotube. Disconnecting the delivery tube from the proximal extension can include breaking the hypotube at the disconnection feature, thereby disconnecting the distal portion of the hypotube from the proximal portion of the hypotube.

The disconnection feature can include circumferential laser cut openings between the distal portion of the hypotube and the proximal portion of the hypotube. Disconnecting the delivery tube from the proximal extension can further include breaking material of the hypotube between the circumferential laser cut openings.

Disconnecting the delivery tube from the proximal extension can further include depressing the handle portion causing the proximal extension to bend to a predetermined bend angle with respect to the proximal end of the delivery tube, thereby causing the hypotube to break.

The disconnection feature can be configured to break apart in response to the proximal extension being bent to the predetermined bend angle.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIGS. 1A, 1B, and 1C are illustrations of an exemplary handle member, according to aspects of the present disclosure.
FIGS. 2A and 2B illustrate a side profile cross-sectional views of an exemplary handle member with a handle portion, delivery tube, and proximal extension, according to aspects of the present disclosure.
FIGS. 3A, 3B, 3C, and 3D illustrate an exemplary handle member with a handle slider, delivery tube, and proximal extension, according to aspects of the present disclosure.
FIG. 4A, 4B, 4C, and 4D illustrate an exemplary handle member with a handle lever, delivery tube, and proximal extension, according to aspects of the present disclosure.
FIG. 5A, 5B, 5C, and 5D illustrate an exemplary handle member with a handle knob, delivery tube, and proximal extension, according to aspects of the present disclosure.
FIGS. 6A, 6B, and 6C illustrate an exemplary delivery system including a delivery tube, proximal end, proximal extension, pull wire, and disconnection feature, according to aspects of the present disclosure.
FIGS. 7A and 7B illustrate a side isometric view and an axial view of an exemplary disconnection feature joining the delivery tube with the proximal extension, according to aspects of the present disclosure.
FIG. 8 illustrates a distal end of the delivery system, including the delivery tube, pull wire, and embolic coil, according to aspects of the present disclosure.
FIG. 9 is a flowchart of an exemplary method of using the delivery system, according to aspects of the present disclosure.

### DETAILED DESCRIPTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the pertinent art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ± 10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

Examples presented herein utilize a handle design to facilitate mechanical deployment of an implant. Examples of implants that can be delivered mechanically include embolic implants, stents, grafts, drug delivery implants, flow diverters, filters, stimulation leads, sensing leads, or other implantable structures deliverable through a microcatheter. In examples, presented herein, according to the present invention, a handle member can be attached to a proximal extension of a delivery tube. The proximal extension and the delivery tube can have a common lumen, with a pull wire disposed within the common lumen. The distal end of the delivery tube can house the implant. The proximal extension can be joined to the delivery tube at a disconnection feature that is configured to break when the proximal extension is bent to a predetermined angle. According to aspects of the disclosure, the handle can be configured to engage to the proximal extension and bend the proximal extension to the predetermined bend angle, thereby causing the disconnection feature to break. The handle can then be manipulated proximally, thereby causing the pull wire and the proximal extension to be translated proximally to release the implant.

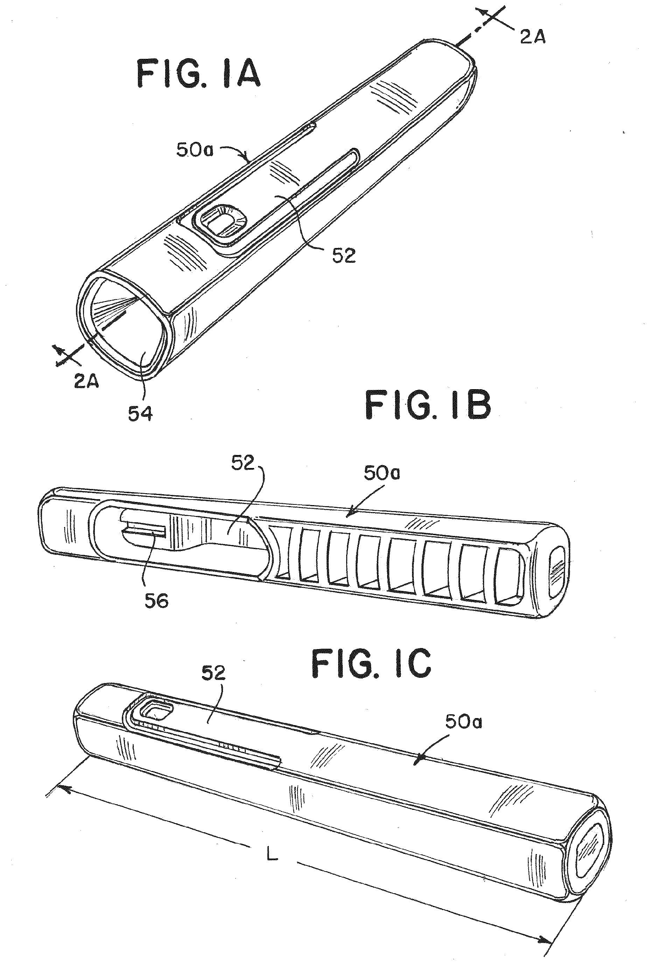

FIGS. 1A through 1C are illustration of an exemplary handle member 50a, according to aspects of the present disclosure. More specifically, FIG. 1A is a top view isometric illustration of handle member 50a from the distal end, FIG. 1B is a bottom isometric view of handle member 50a from the proximal end, and FIG. 1C is a top view isometric illustration of handle member 50a from the proximal end. The exemplary handle member 50a can include a handle portion 52, an aperture 54, and a handle channel 56. The handle member may have a length L extending from a proximal end of the handle member 50a to the distal end of the handle member 50a. According to some aspects of the present disclosure, the length L of handle member 50a can be approximately between 10 cm and 20 cm.

The handle channel 56, found on the bottom of handle member 50a, can be configured to engage with proximal extension 120a and can have a length sufficient to engage to the length of proximal extension 120a. Aperture 54 has a diameter sufficient to fit proximal extension 120a therethrough. Proximal extension 120a is configured to attach to pass through aperture 54 when engaging to handle member 50a at handle channel 56.

Handle portion can be formed in a tongue-shape that can be created by removing a layer of handle member 50a material in a "U" shape, thereby forming a tongue shape of exemplary handle portion 52. Handle portion 52 can be pushed inwards (e.g., towards the bottom surface of handle member 50a), pressing the bottom surface of handle portion 52 against the top surface of handle channel 56.

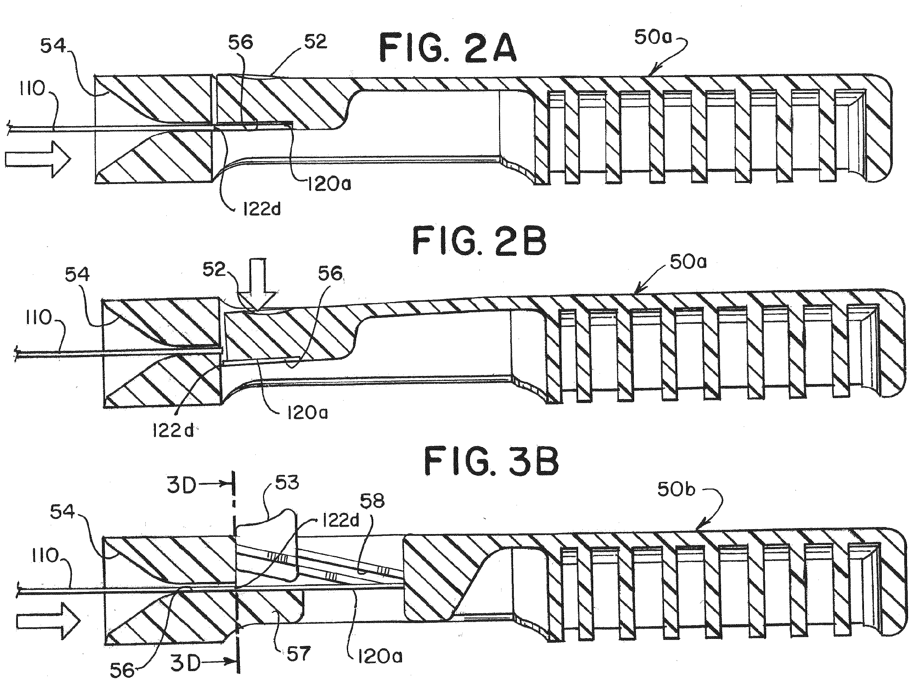

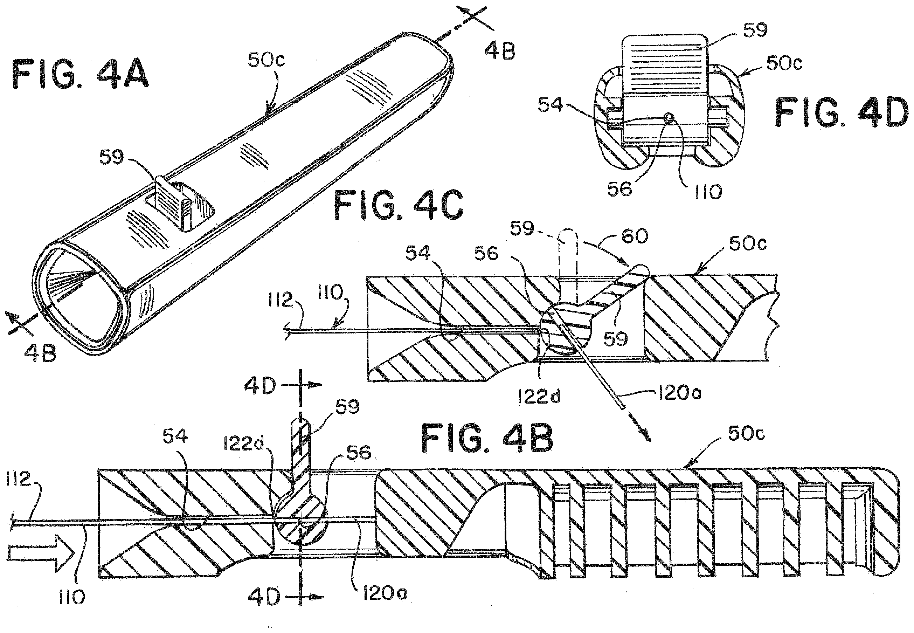

FIG. 2A shows a side profile view of an exemplary handle member 50a and delivery tube 110 being engaged to handle member 50a. Delivery tube 110 can be positioned through aperture 54 of handle member 50a during the preparation of implantation system 100a for implantation. As shown, proximal extension 120a is located on proximal end of delivery tube 110. Proximal extension 120a may have a length portion that can interface with handle channel 56 of handle member 50a. Handle channel 56 may have a height (e.g., measured from top to bottom of the handle channel 56) that is sufficient to house proximal extension 120a without compressing the proximal extension 120a or causing excessive friction at the interface between handle channel 56 and proximal extension 120a. Accordingly, the height of handle channel 56 may be slightly larger than the diameter of proximal extension 120a, for example between approximately 5% and 10% larger than the diameter of proximal extension 120a. Connecting proximal end 112 of delivery tube 110 to proximal extension 120a may be a disconnection feature 122d. As described in more detail with respect to FIG. 4, disconnection feature 122d can be a weakened portion of delivery tube 110 at the interface between proximal end 112 of delivery tube 110 and the proximal extension 120a.

FIG. 2B shows a side profile view of an exemplary handle member 50a with a handle portion 52 applying a compressive force to the proximal extension 120a to facilitate deployment of an implant on the distal end 114 of delivery tube 110. Once delivery tube is moved proximally through aperture 54 to engage proximal extension 120a into handle channel 56. When an operator of implantation system 100a is ready to deliver an implant into a patient, the operator may apply a compressive force to handle portion 52 as indicated by the arrow. The applied force on handle portion 52 is configured to bend handle portion 52, thereby compressing the bottom of surface of handle portion 52 against handle channel 56 and compressing the proximal extension 120a therebetween. Handle channel 56 and handle portion 52 may bend at a predetermined angle with respect to the proximal end 112 of delivery tube 110 in order to allow proximal extension 120a to separate from proximal end 112 of delivery tube 110 at the disconnection feature 122d. According to some embodiments, the predetermined angle can be between approximately 45 degrees to 75 degrees, and in a preferred embodiment, the predetermined bend angle can be approximately 60 degrees. An operator of implantation system 100a can apply a predetermined force to handle portion 52 in order to bend the proximal extension 120a to the predetermined bend angle. According to some embodiments, the force required to bend proximal extension 120a to the predetermined bend angle with respect to proximal end 112 of delivery tube 110 may be in the range of approximately 30 gram-force to approximately 60 gram-force.

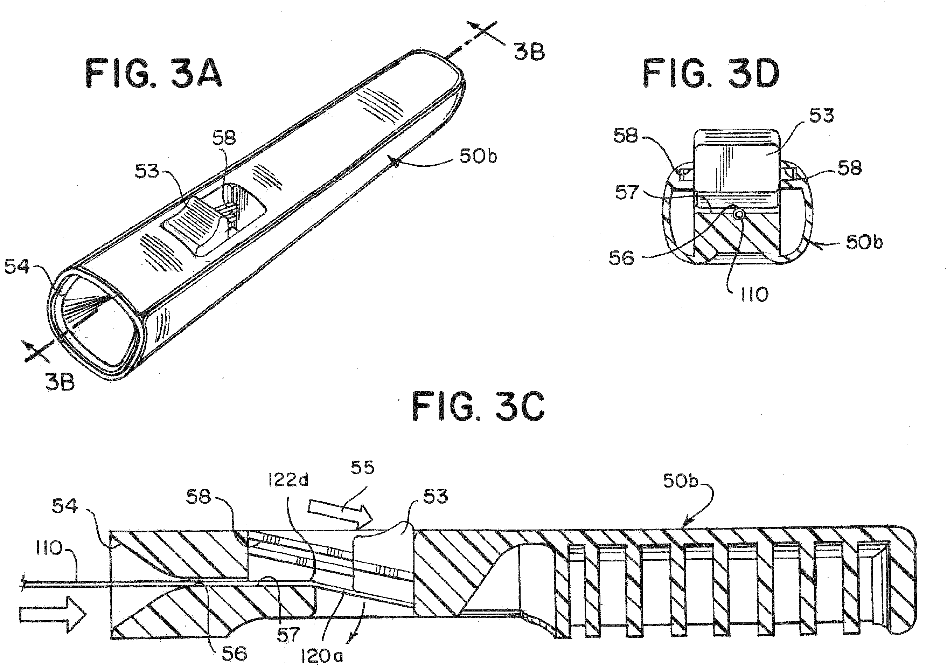

FIG. 3A shows an isometric view of an exemplary handle member 50b. As shown in FIG. 3A, handle member 50b can include a handle slider 53, aperture 54, and a handle channel 56. The function of handle member 50b is discussed in more detail with respect to FIGS. 3B-3D.

FIG. 3B shows an exemplary handle member 50b with a delivery tube 110 interfacing with handle member 50b at handle channel 56 through aperture 54. Exemplary handle member 50b is similar to handle member 50a, except in place of handle portion 52 configured to be compressed against handle channel 56 in order to bend the proximal extension 120a to the predetermined angle with respect to proximal end 112 of delivery tube 110, handle member 50b may provide a handle slider 53 and slider path 58. Additionally, handle member 50b includes a support member 57 that holds proximal end 112 of delivery tube 110 in place in relation to the proximal extension 120a as handle slider is slid proximally along slider path 58. Handle slider 53 can be a member configured to be slid proximally by an operator of delivery system 100a along the slider path 58. Slider path 58 may be angled with respect to delivery tube 110 such that as handle slider 53 is slid proximally (e.g., towards the proximal end of handle member 50b), handle slider is translated towards the bottom surface of handle member 50b, thereby putting pressure on proximal extension 120a and bending proximal extension 120a to the predetermined angle, allowing the proximal extension 120a to become detached from proximal end 112 at disconnection feature 122d.

FIG. 3C shows exemplary handle member 50b at the moment that handle slider 53 is slid proximally 55 along slider path 58 thereby disconnecting proximal extension 120a from proximal end 112 of delivery tube 110 at disconnection feature 122d. As discussed with respect to FIG. 3B, handle slider 53 is slid proximally along slider path 58, thereby bending proximal extension 120a to the angle with respect to proximal end 112 of delivery tube 110, thereby separating proximal extension 120a from the proximal end 112 at the disconnection feature 122d.

FIG. 3D illustrates an axial view of handle member 50b, according to aspects of the present disclosure. As shown in FIG. 3D, handle member 50b includes handle slider 53 that is configured to slide along slider path 58. Handle member 50b is configured to receive a proximal extension 120a of delivery tube 110 through aperture 54. The delivery tube is supported by a support member 57, which fixes the proximal end 112 of delivery tube 110 in place in relation to the proximal extension 120a as handle slider is slid proximally 55 along slider path 58.

FIG. 4A shows an isometric view of an exemplary handle member 50c. As shown in FIG. 4A, handle member 50c can include a handle lever 59. The function of handle member 50c is discussed in more detail with respect to FIGS. 4B-4D.

FIG. 4B shows an exemplary handle member 50c with a delivery tube 110 interfacing with handle member 50b at handle channel 56 through aperture 54. Exemplary handle member 50c is similar to handle members 50a and 50b, except in place of handle portion 52 or handle slider 53, handle member 50c may include a handle lever 59 that is configured to pivot and translate in an arc 60, thereby moving the position of handle channel 56 through which the delivery tube 110 interfaces with handle member 50c. Handle member 50c also includes a support member 57 that holds proximal end 112 of delivery tube 110 in place in relation to the pivoting of handle channel 56 caused by the arc travel 60 of handle lever 59. Handle lever 59 can be a member configured to be pivoted proximally by an operator of delivery system 100a. As handle lever 59 completes the travel arc 60, the handle channel 56 pivots with respect to proximal end 112 of delivery tube 110, putting pressure on proximal extension 120a and bending proximal extension 120a to the predetermined angle, allowing the proximal extension 120a to become detached from the proximal end 112 at disconnection feature 122d.

FIG. 4C shows exemplary handle member 50c at the moment that the handle lever 59 is pivoted along its travel arc 60 thereby disconnecting proximal extension 120a from proximal end 112 of delivery tube 110 at disconnection feature 122d. As discussed with respect to FIG. 4B, as handle lever 59 completes the travel arc 60, the handle channel 56 pivots with respect to proximal end 112 of delivery tube 110, putting pressure on proximal extension 120a and bending proximal extension 120a to the predetermined angle, allowing the proximal extension 120a to become detached from the proximal end 112 at disconnection feature 122d.

FIG. 4D illustrates an axial view of handle member 50c, according to aspects of the present disclosure. As shown in FIG. 4D, handle member 50c includes the handle lever 59 that is configured to pivot and travel in arc 60 thereby disconnecting proximal extension 120 from proximal end 112 at disconnection feature 122d. Deliver tube 110 is shown interfacing with handle member 50c through aperture 54.

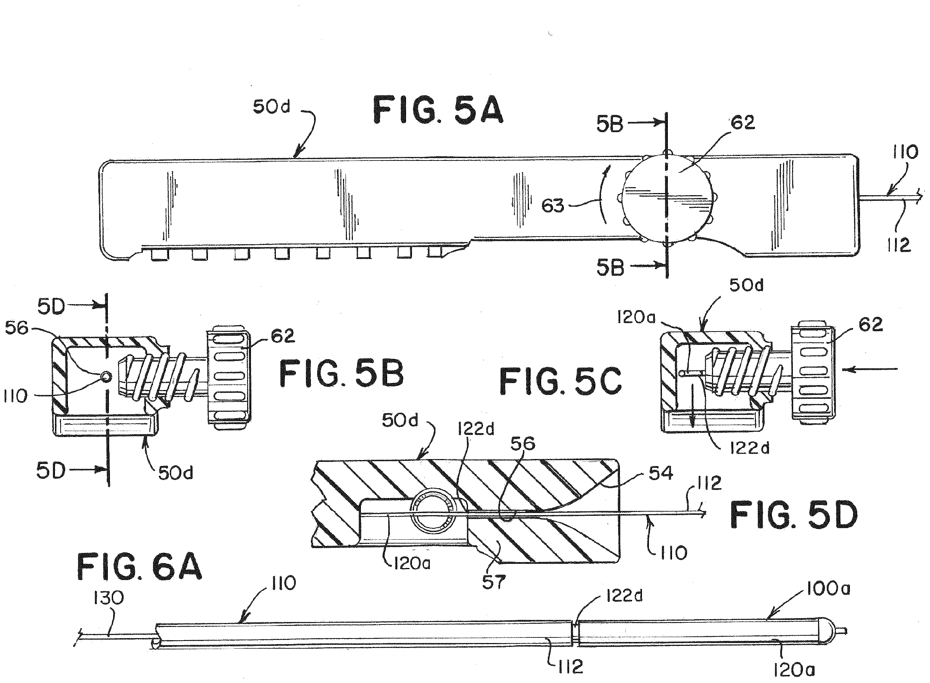

FIG. 5A shows a top-down view of an exemplary handle member 50d. As shown in FIG. 5A, handle member 50d can include a handle knob 62. Handle knob 62 is configured to rotate 63. Exemplary handle member 50d is similar to handle members 50a, 50b, and 50c, except that in place of handle portion 52, handle slider 53, or handle lever 59, handle member 50d may include handle knob 62 that is configured to rotate 63, thereby screwing knob 62 down until it interferes with proximal extension 122a of delivery tube 110. The function of handle member 50d is discussed in more detail with respect to FIGS. 5B-5D.

FIGS. 5B and 5C shows a cut-away view of exemplary handle member 50d at the moment that handle knob 62 is rotated 63 to interfere with proximal extension 120a, causing the proximal extension 120a to bend to a predetermined angle with respect to proximal end 112 of delivery tube 110, thereby causing proximal extension 120a to disconnect from proximal end 112 of delivery tube 110.

FIG. 5D shows a cutaway side profile view of exemplary handle member 50d. As can be seen in FIG. 5D, handle member 50d includes a support member 57 that holds proximal end 112 of delivery tube 110 in place in relation to the proximal extension 120a as handle knob 52 is rotated 63 to cause proximal extension 120a to bend to a predetermined angle with respect to proximal end 112 of delivery tube. In addition, proximal extension 120a interfaces with handle member 50d at handle channel through aperture 54.

FIGS. 6A through 6C are illustrations of exemplary implantation system 100a which has a proximal extension 120a. For illustration purposes only, implantation system 110a is shown without handle member 50, which may be used in order to detach proximal extension 120a from proximal end 112. The implantation system 100a can have an embolic implant 140 such as an embolic coil, embolic braid, or other such implant for filling an aneurysm sac, a delivery tube 110 for delivering the embolic implant 140 to a treatment site, and a pull wire 130 disposed within the delivery tube 110 that can be pulled proximally to deploy the embolic implant 140. When handle member 50 engages proximal extension 120a, proximal extension 120a, pull wire 130, and handle member 50 can be translated proximally as a single unit in order to deploy the embolic implant 140.

FIG. 6A illustrates the system 100a in a delivery configuration. While the system 100a is in the delivery configuration, the delivery tube 110 can be introduced into the body of a patient and the embolic implant 140 can be positioned at a treatment site.

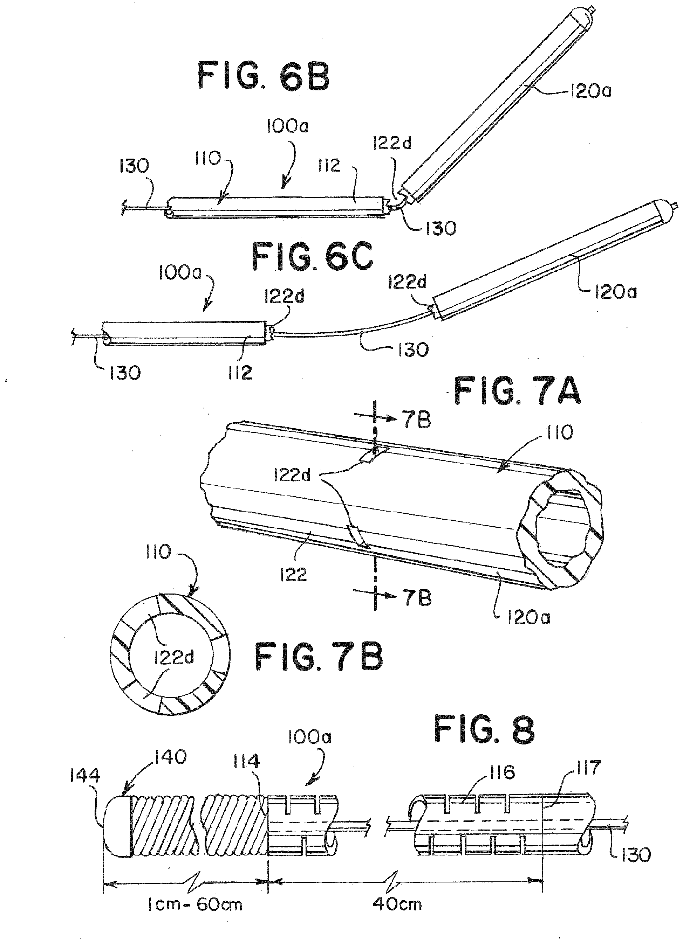

FIG. 6B illustrates the system 100a at step in preparation for deploying the embolic implant 140. The system 100a can include a disconnection feature 122d between the delivery tube 110 and the proximal extension 120a that can be manipulated to separate the delivery tube 110 from the proximal extension 120a (e.g., by depressing the handle portion 52 of handle member 50a and/or sliding the handle slider 53 proximally along slider path 58 in handle member 50b). As illustrated, the disconnection feature 122d can be broken by bending the proximal extension 120a to the predetermined angle in relation to the delivery tube 110.

FIG. 6C illustrates the proximal extension 120a being pulled proximally to thereby pull the pull wire 130 proximally and release the embolic implant 140.

FIGS. 7A and 7B illustrate a side isometric view and an axial view of the disconnection feature 122d. As illustrated, the delivery tube 110 and the proximal extension 120a be formed from a contiguous hypotube. The disconnection feature 122d can include laser-cut features in the hypotube that are sized and spaced so that stress is concentrated on mater between the laser-cut features so that the hypotube breaks at the disconnection feature 122d when bent as illustrated in FIG. 6B. The laser-cut features can be alternatively sized, shaped, and otherwise configured to concentrate stress on hypotube material so that the delivery tube 110 breaks at a predetermined position in response to bending. According to some embodiments, disconnection feature 122d can be a portion of the hypotube that is welded together circumferentially. The weld can be a weakened portion of the hypotube in relation to the rest of the hypotube such that, when proximal extension is bent in relation to delivery tube, the hypotube can break at the disconnection feature 122d.

FIG. 8 is an illustration of an exemplary implantation system 100a. The implantation system 100a can have an embolic implant 140 such as an embolic coil, embolic braid, or other such implant for filling an aneurysm sac, a delivery tube 110 for delivering the embolic implant 140 to a treatment site, a pull wire 130 disposed within the delivery tube that can be pulled proximally to deploy the embolic implant 140, handle member 50 (not shown) positioned at a proximal end of the delivery tube 110 and attached to proximal extension 120a that can be bent to a predetermined angle with respect to delivery tube 110 thereby separating proximal extension 120a from delivery tube 110 and allowing the handle member 50, proximal extension 120a and pull wire 130 to be translated proximally to deploy embolic implant 140.

The delivery tube 110 can have a soft section 116 positioned near a distal end 114 of the delivery tube 110 that has a greater flexibility than the remainder (proximal portion) 112 of the delivery tube 110. The embolic implant 140 can be detachably attached to a distal end 114 of the delivery tube 114. The soft section 116 can be designed to allow greater control and stability of the distal end 114 of the delivery tube 110 during implantation and deployment of the embolic implant 140. The soft section 116 can have laser cut notches or groves, and/or the soft section 116 can be made of a more flexible material compared to the proximal end 112 of the delivery tube 110.

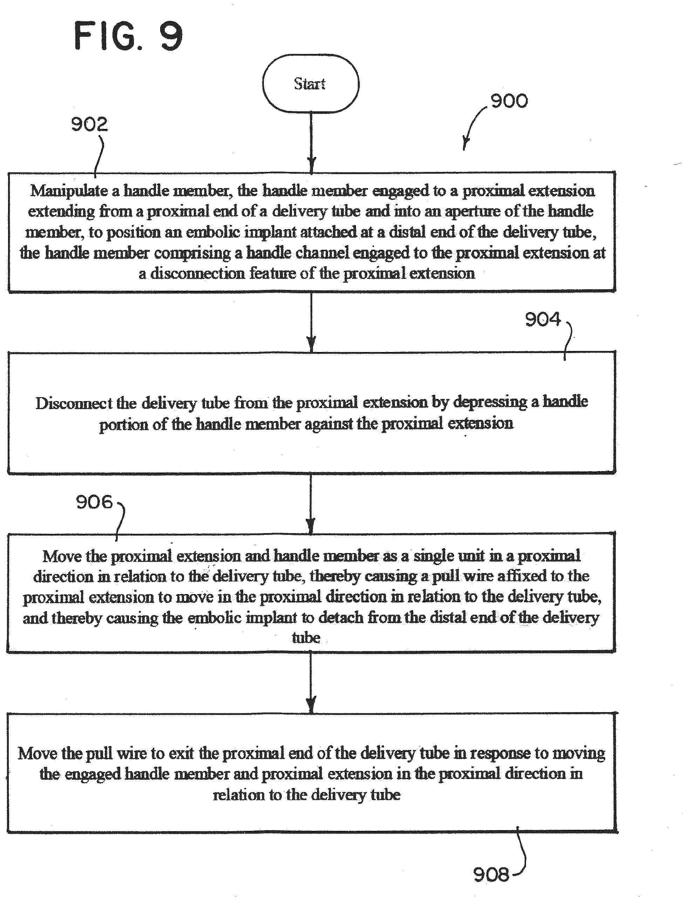

FIG. 9 is a flowchart of an exemplary method of using the delivery system, according to aspects of the present disclosure. In block 902, the method may include manipulating a handle member 50a. The handle member 50a can be engaged to a proximal extension 120a that extends from a proximal end 112 of delivery tube 110 and extends into an aperture 54 of the handle member 50a. The handle member 50a can be manipulated to position an embolic implant 140 into position at a treatment site. The embolic implant can be attached at a distal end 114 of the delivery tube 110. The proximal extension 120a can be engaged to the handle member 50a via a handle channel 56 at a disconnection feature 122d of the proximal extension 120a.

In block 904, the method can include disconnecting the delivery tube 110 from the proximal extension 120a by depressing a handle portion 52 of the handle member 50a against the proximal extension 120a. According to some embodiments, disconnecting the delivery tube 110 from the proximal extension 120a can further include depressing the handle portion 52 to cause the proximal extension 120a to bend to a predetermined bend angle with respect to the proximal end 112 of the delivery tube 110 to cause the hypotube to break at the disconnection feature 122d. According to some embodiments, the disconnection feature 122d can be configured to break apart in response to the proximal extension 120a being bend to the predetermined bend angle.

In block 906, the method can include moving the proximal extension 120a and handle member 50a as a single unit in a proximal direction in relation to the delivery tube 110 to thereby cause a pull wire 130 that is affixed to the proximal extension 120a to move in the proximal direction in relation to the delivery tube 110. The proximal movement of pull wire 130 can cause the embolic implant 140 to detach from the distal end 114 of the delivery tube at the treatment site.

In optional block 908, the method can include moving the pull wire 130 to exit the proximal end 112 of the delivery tube 110 in response to moving the engaged handle member 50a and proximal extension 120a in the proximal direction in relation to the delivery tube 110.

In some embodiments, the delivery tube 110 includes a distal portion of a hypotube, and the proximal extension 120a can include a proximal portion of the hypotube. Disconnecting the delivery tube 110 from the proximal extension 120 can further include breaking the hypotube at the disconnection feature 122d to thereby disconnect the distal portion of the hypotube from the proximal portion of the hypotube.

In some embodiments, the disconnection feature 122d can include circumferential laser cut openings between the distal portion of the hypotube and the proximal portion of the hypotube. Disconnecting the delivery tube 110 from the proximal extension 120a can further include breaking material of the hypotube between the circumferential laser cut openings.

The descriptions contained herein are examples of embodiments of the invention and are not intended in any way to limit the scope of the invention. As described herein, the invention contemplates many variations and modifications of the implantation system and associated methods, including alternative geometries of system components, alternative materials, additional or alternative method steps, etc. Modifications apparent to those skilled in the pertinent art are intended to be within the scope of the claims which follow.

Aspects of the invention:
1. A method for treating an aneurysm comprising:
   manipulating a handle member, the handle member engaged to a proximal extension extending from a proximal end of a delivery tube and into an aperture of the handle member, to position an embolic implant attached at a distal end of the delivery tube, the handle member comprising a handle channel engaged to the proximal extension at a disconnection feature of the proximal extension;
   disconnecting the delivery tube from the proximal extension by depressing a handle portion of the handle member against the proximal extension; and
   moving the proximal extension and handle member as a single unit in a proximal direction in relation to the delivery tube, thereby causing a pull wire affixed to the proximal extension to move in the proximal direction in relation to the delivery tube, and thereby causing the embolic implant to detach from the distal end of the delivery tube.
2. The method of aspect 1, further comprising:
   moving the pull wire to exit the proximal end of the delivery tube in response to moving the engaged handle member and proximal extension in the proximal direction in relation to the delivery tube.
3. The method of aspect 1,
   wherein the delivery tube comprises a distal portion of a hypotube,
   wherein the proximal extension comprises a proximal portion of the hypotube, and
   wherein disconnecting the delivery tube from the proximal extension further comprises breaking the hypotube at the disconnection feature thereby disconnecting the distal portion of the hypotube from the proximal portion of the hypotube.
4. The method of aspect 3,
   wherein the disconnection feature comprises circumferential laser cut openings between the distal portion of the hypotube and the proximal portion of the hypotube, and
   wherein disconnecting the delivery tube from the proximal extension further comprises breaking material of the hypotube between the circumferential laser cut openings.
5. The method of aspect 3, wherein disconnecting the delivery tube from the proximal extension further comprises depressing the handle portion causing the proximal extension to bend to a predetermined bend angle with respect to the proximal end of the delivery tube, thereby causing the hypotube to break.
6. The method of aspect 5, wherein the disconnection feature is configured to break apart in response to the proximal extension being bent to the predetermined bend angle.

## Claims

1. A system for delivery of an embolic implant, comprising:
a delivery tube comprising a lumen therethrough, a proximal end, and a distal end;
a proximal extension positioned approximate the proximal end of the delivery tube comprising a proximal end and a distal end, the proximal end disposed within a handle channel of a handle member through an aperture of the handle member;
the handle member comprising a handle portion that, when depressed, is configured to selectively engage the handle member to the proximal extension and bend the proximal extension to a predetermined bend angle with respect to the proximal end of the delivery tube;
an elongated member disposed within the lumen of the delivery tube;
an embolic coil attached to the delivery tube approximate the distal end of the delivery tube and configured to detach from the delivery tube upon proximal translation of the elongated member; and
a disconnection feature fixing a position of the proximal extension in relation to the delivery tube and configured to break at the predetermined bend angle to allow proximal translation of the proximal extension and elongated member in relation to the delivery tube.

2. The system of claim 1, wherein the predetermined bend angle that causes the disconnection feature to break apart comprises approximately 60 degrees between the proximal extension and the proximal end of the delivery tube.

3. The system of claim 1,
wherein the proximal extension is detachable from the delivery tube in response to the disconnection feature breaking,
wherein the proximal extension and handle member, when selectively engaged, are configured to translate proximally as a single unit, and
wherein the elongated member is movable to exit the proximal end of the delivery tube in response to the proximal translation of the proximal extension.

4. The system of claim 1, wherein the delivery tube comprises a distal portion of a hypotube, the proximal extension comprises a proximal portion of the hypotube, and the disconnection feature is disposed between the distal portion of the hypotube and the proximal portion of the hypotube.

5. The system of claim 4, wherein the disconnection feature comprises circumferential laser cut openings in the hypotube.

6. The system of claim 4, wherein the disconnection feature is configured to cause the hypotube to break at the disconnection feature when the handle portion is depressed and bends the proximal extension to the predetermined bend angle.

7. The system of claim 1,
wherein the proximal extension comprises a tube comprising a lumen therethrough, and
wherein the elongated member is at least partially disposed within the lumen of the tube of the proximal extension and at least partially disposed within the lumen of the delivery tube.

8. An embolic implantation assembly comprising:
a delivery tube comprising a lumen therethrough, a proximal end, and a distal end;
a proximal extension extending proximally from the proximal end of the delivery tube, the proximal extension configured to pass through an aperture of a handle member;
the handle member configured to selectively engage the proximal extension at a handle channel of the handle member, the handle member comprising a handle slider that is configured to slide proximally along a slider path thereby selectively engaging the handle member to the proximal extension and bending the proximal extension to a predetermined bend angle with respect to the proximal end of the delivery tube;
a disconnection feature disposed between the proximal extension and the proximal end of the delivery tube and joining the proximal extension to the delivery tube, the disconnection feature, when in a closed configuration, having a position fixed in relation to the delivery tube, the disconnection feature configured to open into an open configuration to allow proximal translation of the proximal extension in relation to the delivery tube responsive to being bent to the predetermined bend angle caused by sliding the handle slider along the slider path;
an embolic implant attached to the delivery tube approximate the distal end of the delivery tube;
a pull wire disposed within the lumen of the delivery tube and configured to move proximally to detach the embolic implant from the delivery tube when the proximal extension is translated proximally in relation to the delivery tube.

9. The assembly of claim 8,
wherein the proximal extension is detachable from the delivery tube at the disconnection feature,
wherein the proximal extension and handle member, when selectively engaged, are configured to translate proximally as a single unit, and
wherein the pull wire is movable to exit the proximal end of the delivery tube in response to the proximal translation of the proximal extension in relation to the delivery tube.

10. The assembly of claim 8, wherein the delivery tube comprises a distal portion of a hypotube, the proximal extension comprises a proximal portion of the hypotube, and the disconnection feature is disposed between the distal portion of the hypotube and the proximal portion of the hypotube.

11. The assembly of claim 10, wherein the disconnection feature comprises circumferential laser cut openings in the hypotube.

12. The assembly of claim 10, wherein the predetermined bend angle that causes the disconnection feature to open into the open configuration comprises approximately 60 degrees between the proximal extension and the proximal end of the delivery tube.

13. The assembly of claim 8,
wherein the proximal extension comprises a tube comprising a lumen therethrough, and
wherein the pull wire is at least partially disposed within the lumen of the tube of the proximal extension and at least partially disposed within the lumen of the delivery tube.

14. The assembly of claim 8, wherein the embolic implant comprises an embolic coil.
